# EUROPÄISCHE PATENTSCHRIFT

(11) **EP 1 718 233 B1**
(45) Veröffentlichungstag und Bekanntmachung des Hinweises auf die Patenterteilung: **11.07.2012**
(21) Anmeldenummer: 05707630.9
(22) Anmeldetag: 25.02.2005
(51) Int. Cl.: A61B 18/14, A61F 7/12, A61M 25/10

(54) **VORRICHTUNG ZUR INTERSTITIELLEN KOAGULATION VON GEWEBE**
DEVICE FOR THE INTERSTITIAL COAGULATION OF TISSUE
DISPOSITIF DE COAGULATION INTERSTITIELLE DE TISSUS

(30) Priorität: 25.02.2004 DE 102004009206; 16.03.2004 DE 102004012813
(43) Veröffentlichungstag der Anmeldung: 08.11.2006
(73) Patentinhaber: ERBE Elektromedizin GmbH, 72072 Tübingen (DE)
(72) Erfinder: EISELE, Florian, 72074 Tübingen (DE)
(74) Vertreter: Bohnenberger, Johannes
(86) Internationale Anmeldenummer: PCT/EP2005/002001
(87) Internationale Veröffentlichungsnummer: WO 2005/079688

(56) Entgegenhaltungen:
- US-A- 5 496 311
- US-A- 5 545 195
- US-A1- 2003 130 572
- US-B1- 6 475 213

## Beschreibung

Die Erfindung betrifft eine Vorrichtung zur interstitiellen Koagulation von Gewebe nach Patentanspruch 1.

In der Hochfrequenzchirurgie, insbesondere im Bereich der endoskopischen Anwendung, sind verschiedene Vorrichtungen bekannt, mit deren Hilfe die elektrische Energie eines Hochfrequenzchirurgiegerätes, also einer HF-Einheit an die zu behandelnden Stellen des Gewebes gebracht werden kann.

Es sind Elektroden Bekannt, welche die Energie direkt, also über den Kontakt mit dem Gewebe applizieren. Dabei besteht das Problem, dass das Gewebe unter Umständen so sehr erhitzt wird, dass es karbonisiert, also vollständig verbrennt.

Ein weiteres Problem bei derartigen Elektroden liegt darin, dass die Elektroden am Gewebe ankleben können. Wird die Elektrode dann entfernt, so reißt das Gewebe auf. Um die Gefahr des Anklebens zu verringern, weisen die Elektroden oftmals eine entsprechende Beschichtung auf. Derartige. Elektroden sind z. B. aus der DE 199 41 105 C2 bekannt.

Die oben beschriebenen Probleme kommen insbesondere bei einer interstitiellen Koagulation, beispielsweise bei der Koagulation eines Leber-Tumors zum Tragen, da bei dieser die Elektrode in das zu behandelnde Gewebe eingebracht, z. B. eingestochen wird und somit vollständig von diesem umgeben ist. Die Gefahr eines Verbrennens des Gewebes und/oder eines Anklebens des Gewebes an der Elektrode ist hier in besonders hohem Maße gegeben. Eine gleichmäßige Devitalisierung ist dann u. a. aufgrund eines hohen Übergangswiderstandes zwischen Elektrode und Gewebe nicht mehr möglich, d. h., das Gewebe wird ungleichmäßig koaguliert.

Problematisch ist es weiterhin, dass sich das Gewebe während der interstitiellen Koagulation zusammenzieht (austrocknet), so dass die Elektrode nicht mehr an diesem anliegt.

Ein in Folge der Austrocknung des Tumors entstehender Spalt zwischen der Elektrode und dem zu behandelnden Gewebe erhöht den Übergangswiderstand zwischen Elektrode und Gewebe derart, dass ein Eintrag elektrischer Energie begrenzt wird. Auch dies führt zu einer ungleichmäßigen Koagulation oder sogar dazu, dass kein Koagulationseffekt eintritt.

Aus der US 6 090 105 A ist z. B. ein Abtragungsgerät bekannt, das eine Einführelektrode zum Einstechen in ein zu behandelndes Gewebe aufweist. Im Inneren der Einführelektrode sind aus dieser herausführbare weitere Elektroden angeordnet, die nach dem Einstechen der Einführelektrode in dem Gewebe platziert werden. Die Elektroden sind aus einer Memory-Legierung ausgebildet und stehen ab einer bestimmten Temperatur unter Spannung. Außerhalb der Einführelektrode streben die Elektroden ihre ursprüngliche Gestalt an und dringen somit während eines Koagulationsvorganges in das sie umgebende Gewebe vor. Bei dem soeben beschriebenen Gerät treten die oben diskutierten Probleme gehäuft auf. Zudem ist es gerade bei Elektrodenanordnungen dieser Art schwierig, ein gleichmäßiges Koagulationsvolumen zu erzielen, weil die Elektroden unkoordiniert, nur in Abhängigkeit ihres Ausdehnungsbestrebens in das Gewebe vordringen.

Aus der US 2003/130572 A1 ist eine Vorrichtung nach dem Oberbegriff des Anspruches 1 bekannt.

Der Erfindung liegt die Aufgabe zugrunde, eine Vorrichtung zur interstitiellen Koagulation von Gewebe dahin gehend weiterzubilden, dass Gewebe mit erhöhter Gleichmäßigkeit behandelbar ist.

Diese Aufgabe wird durch eine Vorrichtung zur interstitiellen Koagulation von Geweben nach Patentanspruch 1 gelöst.

Die Aufgabe wird durch eine Vorrichtung zur interstitiellen Koagulation von Geweben gelöst, die mindestens eine Elektrode aufweist, über die ein HF-Koagulationsstrom in das Gewebe einleitbar ist. Die Elektrode ist als dreidimensionaler, auf verschiedene Aufweitungszustände aufweitbarer Körper derart ausgebildet oder auf einem derartigen Körper derart angebracht, dass die Elektrode durch kontinuierliches oder schrittweises Aufweiten des Körpers mit dem Gewebe während des Koagulierens in ständigem elektrischen Kontakt haltbar ist. Weiterhin ist eine Steuerungseinrichtung zum Steuern des Aufweitungszustandes des Körpers in Abhängigkeit vom Koagulationsstrom vorgesehen. Die Steuerungseinrichtung ist in einem HF-Chirurgiegerät ausgebildet oder aber als externes Bauteil vorgesehen. Des Weiteren ist die Steuerungseinrichtung der Vorrichtung, also im Prinzip dem elektrochirurgischen Instrument, zugeordnet und so ausgebildet, dass sie diverse, den Koagulationsgrad wiedergebende Parameter, hier den Koagulationsstrom detektiert. So lässt sich z. B. die Zunahme des Übergangswiderstandes aufgrund oben beschriebener Spaltbildung zwischen Elektrode und Gewebe über das Absinken der Koagulationsstromstärke erfassen. Die Steuerungseinrichtung ist weiterhin dazu ausgebildet, den Körper nun aufgrund des detektierten Parameters, also z. B. aufgrund der detektierten Stromstärke derart anzusteuern, dass dieser seinen Aufweitungsszustand in Abhängigkeit des detektierten Parameters verändert. Der Körper wird also in diesem Falle bis zur Überwindung des Spalts aufgeweitet, so dass Elektrode und Gewebe in, dem weiteren Koagulationsvorgang förderlichen, elektrischen Kontakt haltbar sind. Damit ist in jeder Phase der Koagulation ein optimales Koagulationsergebnis gewährleistet.

Ein wesentlicher Punkt der Erfindung liegt darin, dass der Körper, d. h. die Elektrode selbst oder ein aufweitbarer Körper, auf dem die Elektrode angeordnet ist, in einem nicht-aufgeweiteten Zustand in das zu behandelnde Gewebe einbringbar ist und während der Koagulation, entsprechend dem Koagulationsgrad, kontrolliert, also von definierten Bedingungen abhängig, aufgeweitet werden kann. Der Körper ist also derart ausgebildet und betätigbar, dass er dem behandelten, in der Regel zurückweichenden Gewebe während der Koagulation kontrolliert folgt, wobei die Aufweitung aufgrund detektierter Parameter, die ein Maß für den Koagulationsgrad darstellen, durchführbar ist. Die kontrollierte Koagulation ermöglicht eine gleichmäßige Gewebedevitalisierung. Die sich immer weiter aufweitende Elektrode zieht das zu behandelnde Gewebe während der Koagulation zudem glatt, so dass bereits durch diesen Aspekt eine gleichmäßige Koagulation gewährleistet ist.

Die Vorrichtung zur interstitiellen Koagulation ist beispielsweise mittels einer zum Einstechen in das zu behandelnde Gewebe geeigneten Einführeinrichtung in das Gewebe einbringbar oder aber die Vorrichtung ist selbst derart ausgebildet, dass ein Einstechen in das Gewebe möglich ist. Die Vorrichtung kann sowohl für den unmittelbaren Einsatz oder für die endoskopische Anwendung ausgebildet sein.

Vorzugsweise ist der Steuerungseinrichtung ein Messwertaufnehmer zugeordnet, der den Koagulationsstrom am Zielgewebe misst und die gemessenen Werte an die Steuerungseinrichtung übermittelt. Eine zuverlässige Stromüberwachung lässt sich z. B. auch mit einem Strommonitor realisieren, wie er aus dem Stand der Technik bekannt ist.

Eine beginnende Spaltbildung lässt sich alternativ über eine Druckmessung bestimmen. Dazu ist die Steuerungseinrichtung, ggf. mit dem Messwertaufnehmer, so ausgebildet, dass beispielsweise der Anpressdruck des Zielgewebes an die Elektrode gemessen wird, wobei bei einem Unterschreiten eines definierten Druckes der Körper derart anzusteuern ist, dass sich dieser zur Wahrung eines geeigneten Abstandes zwischen Elektrode und Gewebe entsprechend aufweitet. Der Messwertaufnehmer ist dazu vorzugsweise als elektronischer Drucksensor ausgebildet. Auch ist es möglich, einen Halleffektsensor zu verwenden, der die Änderung eines Magnetfeldes in Abhängigkeit von der Auslenkung der Elektrode bestimmt.

Bei einer simultanen Messung des Koagulationsstromes und des Druckes, der auf die Elektrode wirkt, kann zudem eine Aussage über einen unerwünschten Verbrennungsgrad des Gewebes oder ggf. über ein Ankleben der Elektrode getroffen werden. Wird ein angemessener Druck bei gleichzeitigem Stromabfall detektiert, muss nämlich davon ausgegangen werden, dass die Stromstärke, nicht aufgrund Spaltbildung zwischen Elektrode und Gewebe absinkt.

In einer bevorzugten Ausführungsform ist vorgesehen, die Steuerungseinrichtung derart anzuordnen und auszubilden, dass eine Stromdichte des Koagulationsstromes zwischen Elektrode und Gewebe einstellbar ist. Der dreidimensionale Körper beschreibt in jedem Aufweitungszustand eine definierte Elektrodenfläche. Bei einer konstanten Stromstärke nimmt eine Stromdichte mit zunehmender Aufweitung des Körpers ab, weil sich die Elektrodenfläche vergrößert. Über die Steuerungseinrichtung ist deshalb die für ein optimales Koagulationsergebnis erforderliche Stromdichte einstellbar und die Stromstärke nachregelbar, so dass auch bei sich vergrößernder Elektrodenfläche zu jedem Zeitpunkt des Koagulationsvorganges ein optimales Koagulationsergebnis gewährleistet ist. Die Einstellung der Stromstärke ist dabei sowohl selbsttätig als auch manuell durchführbar.

Die erfasste Stromdichte und ein ggf. erforderliches Nachregulieren des Koagulationsstromes stellen ein Maß für den Aufweitungszustand des Körpers und damit auch ein Maß für den Koagulationsgrad dar. Unterschreitet die Stromdichte einen vorbestimmten Schwellenwert, so ist die für einen definierten Zielgewebebereich vorgesehene Koagulation abgeschlossen. Ein weiteres Koagulieren würden dann ein Aufweiten des Körpers voraussetzen, wobei die erneute Koagulation wiederum über eine Veränderung der Stromdichte beobachtbar wäre. Die Einstellung einer entsprechenden Stromstärke lässt sich manuell durchführen. Es ist jedoch möglich, die Steuerungseinrichtung derart auszubilden, dass die Stromstärke selbsttätig eingestellt wird.

Vorzugsweise ist die Steuerungseinrichtung derart ausgebildet, dass die Stromdichte unabhängig vom Aufweitungszustand einstellbar ist. Damit stehen für unterschiedliche Gewebearten oder Koagulationsstadien unterschiedliche Koagulationsstärken zur Verfügung. Tritt beispielsweise eine unerwartete Blutung auf, so kann eine Erhöhung der Stromdichte eine stärkere Koagulation bewirken.

Eine erfindungsgemäße Lösung sieht vor, dass Messeinrichtungen zum Feststellen des Aufweitungszustandes des Körpers vorgesehen sind. Damit lässt sich zu jedem Zeitpunkt auf das Koagulationsmaß schließen, wenn die Aufweitung selbsttätig gesteuert ist. Der Aufweitungszustand kann beispielsweise über ein optische Anzeige ausgegeben werden. Bei einer manuell durchzuführenden Aufweitung des Körpers kann der Operateur über die Anzeige eine entsprechende Nachregulierung durchführen. Die Messeinrichtungen können über den oben bereits angeführten Messwertaufnehmer realisiert sein.

Vorzugsweise umfasst die Elektrode der Vorrichtung eine mindestens teilweise fluiddurchlässige Behandlungselektrode, die mit einem Abschnitt des Gewebes in Berührung bringbar ist, eine Zufuhreinrichtung für Flüssigkeit, über die eine elektrisch leitfähige Flüssigkeit der Behandlungselektrode zuführbar ist und eine Stromzufuhreinrichtung zum Zuführen des HF-Koagulationsstromes zur Behandlungselektrode derart, dass von der Behandlungselektrode durchgelassener Flüssigkeit der HF-Behandlungsstrom zuführbar ist. Ein solchermaßen ausgebildetes Instrument weist also vorzugsweise einen aufweitbaren Hohlkörper auf, der in einem nicht-aufgeweiteten Zustand in das zu behandelnde Gewebe einbringbar ist und während der Koagulation durch seine Aufweitung dem zurückweichenden, koagulierten Gewebe folgen kann. Dies hat neben dem Glattziehen des zu behandelnden Gewebes den Vorteil, dass gleichzeitig ein Ankleben der Elektrode an dem Gewebe durch die Stromleitung mittels der Flüssigkeit wirksam verhindert wird. Die elektrische Leitfähigkeit der Flüssigkeit bzw. der Lösung (z. B. Ringer-Lösung oder reine Kochsalz-Lösung) dient dazu, den HF-Strom an die zu behandelnde Gewebestelle zu bringen. Gleichzeitig kühlt die Lösung das Gewebe während der Behandlung, so dass es kaum über den Siedepunkt der Lösung erwärmt wird.

In einer bevorzugten Ausführungsform umfasst die Behandlungselektrode ein elastisch dehnbares oder auffaltbares Flächenelement, auf dessen, dem Gewebe gegenüberliegender Innenseite ein mit einem Innendruck beaufschlagbarer Innenraum derart angeordnet ist, dass das Flächenelement durch Erhöhung des Innendruckes dehnbar ist. Das Flächenelement ermöglicht eine gleichmäßige Kontaktierung des zu behandelnden Gewebes und erleichtert die Aufrechterhaltung des elektrischen Kontaktes zwischen Elektrode und Gewebe, so dass eine gleichmäßige Devitalisierung erzielbar ist. Die während der Koagulation gewünschte Aufweitung des Körpers, hier des Flächenelements, wird durch die Erhöhung des Innendrucks im Innenraum auf einfachste Weise durchgeführt.

Vorzugsweise ist das Flächenelement ring- oder kugelförmig ausgebildet. Die Behandlungselektrode kann dann z. B. in Form eines Ballon-Hohlkatheters aufgebaut sein, also eine Form aufweisen, die der Operateur bereits kennt.

Bei einer Ausführungsform der Erfindung wird der Innenraum mit der elektrisch leitfähigen Flüssigkeit befüllt. Bei dieser Anordnung bildet also die elektrisch leitfähige Flüssigkeit gleichzeitig ein Medium, mit welchem der Innendruck zum Dehnen/Aufblasen des Flächenelementes erzeugt wird. Die Behandlungselektrode ist hierbei derart aufgebaut, dass sie der elektrisch leitfähigen Flüssigkeit einen hinreichend hohen Strömungswiderstand entgegensetzt, so dass einerseits ein Innendruck aufgebaut werden kann, andererseits eine Menge von Flüssigkeit austritt, welche zur Sicherstellung des elektrischen Kontaktes ausreicht. Hierfür kann die Behandlungselektrode eine entsprechend ausgebildete Folie, ein Flies oder ein Gewebe umfassen, welches den genannten Strömungswiderstand aufweist. In jedem Fall ist es von Vorteil, wenn die Behandlungselektrode im Wesentlichen aus einem temperaturbeständigen Material, insbesondere einem TetraFluor-Ethylen-Material aufgebaut ist.

Vorzugsweise umfasst die elektrisch leitfähige Flüssigkeit Polyvinylpyrrolidon (PVP), ein Tensid oder dergleichen Mittel zur Veränderung der Viskosität der elektrisch leitfähigen Flüssigkeit, welches für die hier durchzuführende Behandlung keine negativen Nebeneffekte aufweist.

Der Einsatz einer durch die dehnbare Elektrode diffundierenden Flüssigkeit schließt nicht aus, dass die Elektrodenfläche selbst auch elektrisch leitfähig ist. Eine mögliche Ausführungsform ist eine Elektrode, die aus einem Elastomer hergestellt ist, das beispielsweise durch Einlagern von Metallpartikeln leitfähig gemacht wurde.

Eine Unabhängigkeit des Innendruckes von dem Druck, mit welchem die Flüssigkeit durch die Behandlungselektrode durchgedrückt wird, lässt sich durch eine Anordnung erzielen, bei welcher der Innenraum einen von der elektrisch leitfähigen Flüssigkeit hydraulisch getrennten, expandierbaren Hilfskörper enthält. Dadurch kann auch bei starker Expansion sichergestellt werden, dass nicht zuviel elektrisch leitfähige Flüssigkeit austritt. Insbesondere kann das Flächenelement derart mehrschichtig aufgebaut sein, dass in einer inneren Schicht Flüssigkeit mit einem erniedrigten Strömungswiderstand in Flächenrichtung und in einer äußeren Schicht senkrecht zur Flächenrichtung leitbar ist. Eine derartige Anordnung ist relativ einfach aufbaubar. Zur Erhöhung der Gleichmäßigkeit des Flüssigkeitsaustrittes wird zwischen der inneren und der äußeren Schicht eine Trennschicht mit einem erhöhten Strömungswiderstand angebracht.

In allen Fällen wird der Strom über einen Leiter mit niedrigem elektrischen Widerstand, z. B. einen Draht möglichst nahe an der Behandlungselektrode in die elektrisch leitende Flüssigkeit eingeleitet.

Um Probleme durch die notwendigerweise im Überschuss austretende elektrisch leitende Flüssigkeit zu vermeiden, wird bei einer bevorzugten Ausführungsform der Erfindung eine Saugeinrichtung zum Absaugen überschüssiger Flüssigkeit vorgesehen.

Das elektrochirurgische Instrument, also die Vorrichtung, kann als monopolares Koagulationsinstrument aufgebaut sein, d. h. nur eine einzige Stromzuführung aufweisen, während das zu behandelnde Gewebe (bzw. der Patient) auf das andere Potential gelegt wird. Bei einer anderen bevorzugten Ausführungsform der Erfindung kann durch Verwendung zweier prinzipiell gleicher Elektroden eine bipolare Anwendung realisiert werden. Das heißt, die Behandlungselektrode ist in mindestens zwei voneinander elektrisch isolierte Abschnitte zur Bildung einer bipolaren Elektrode ausgebildet, so dass am Patienten keine Neutralelektrode angelegt werden muss.

Vorzugsweise ist die Elektrode derart ausgebildet, dass sie mit einem Schneidstrom beaufschlagbar ist. Dadurch kann die Gefahr des Freisetzens von Krebszellen beim Einbringen der Elektrode in einen Tumor verringert werden.

Weitere Ausführungsformen der Erfindung ergeben sich aus den Unteransprüchen.

Nachfolgend wird die Erfindung anhand von Ausführungsbeispielen beschrieben, die anhand der Abbildungen näher erläutert werden. Hierbei zeigen
- Fig. 1: ein funktionales Blockschaltbild, das eine HF-Chirurgieanordnung mit einer Vorrichtung für eine interstitielle Koagulation zeigt;
- Fig. 2: eine erste Ausführungsform der Vorrichtung in einer teilgeschnittenen, perspektivischen Darstellung;
- Fig. 3: eine zweite Ausführungsform der Vorrichtung im Längsschnitt;
- Fig. 4: eine dritte Ausführungsform der Vorrichtung im Längsschnitt und
- Fig. 5: eine vergrößerte Schnittdarstellung des Bereiches V aus Fig. 4.

In der nachfolgenden Beschreibung werden für gleiche und gleich wirkende Teile dieselben Bezugsziffern verwendet.

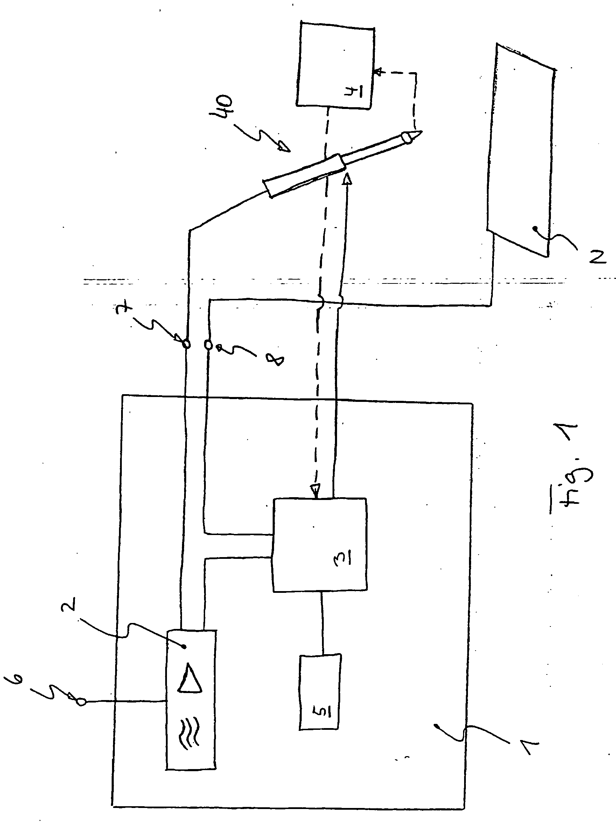

In Fig. 1 ist ein funktionales Blockschaltbild abgebildet, das eine HF-Chirurgieanordnung mit einer Vorrichtung für eine interstitielle Koagulation zeigt. Dabei sind schematisch die für die Erläuterung der Erfindung wesentlichen Komponenten einer HF-Chirurgieanordnung mit einer Vorrichtung 40 zur interstitiellen Koagulation gezeigt.

Bei monopolaren Anordnungen wird ein von einem HF-Generator 2 an ein elektrochirurgisches Instrument 40 (hier die Vorrichtung zur interstitiellen Koagulation) zugeführter HF-Strom in ein zu behandelndes Gewebe über eine differente Elektrode 10, also eine monopolare Koagulationselektrode, appliziert, wobei der Strompfad durch den Körper eines Patienten bis zu der indifferenten Neutralelektrode N führt und von dieser zurück zum HF Generator 2. Bipolare Anordnungen benötigen keine. Neutralelektrode, weil der Strompfad zwischen zwei Elektroden eines elektrochirurgischen Instruments verläuft.

Ein HF-Chirurgiegerät 1 weist einen Eingangsanschluss 6 zum Anschließen von Finger- und/oder Fußschalter aufweisenden Schalteinrichtungen (nicht gezeigt) auf. Über diese Schalteinrichtungen wird ein Aktivieren und/oder Deaktivieren des HF-Stromes ermöglicht. Die Schalteinrichtungen lassen sich hier vorzugsweise über eine Computeranordnung realisieren. Ausgangsseitig sind an dem HF-Chirurgiegerät 1 ein erster Ausgangsanschluss 7 und ein zweiter Ausgangsanschluss 8 vorgesehen, über die das monopolare Koagulationsinstrument 40 mit der dazugehörigen Neutralelektrode N anschließbar ist. Auch ein bipolares elektrochirurgisches Instrument (nicht gezeigt) lässt sich an dem HF-Chirurgiegerät 1 anschließen. Bei der praktischen Ausführung eines HF-Chirurgiegerätes sind zumeist unterschiedliche Anschlüsse für mono- oder bipolare Elektrodenanordnungen vorgesehen. Die Neutralelektrode N ist schematisch dargestellt und bedeckt in der praktischen Anwendung vollständig einen Körperabschnitt eines Patienten.

Kernstück des HF-Chirurgiegerätes 1 ist der steuerbare HF-Generator 2 zum Erzeugen einer HF-Spannung und zum Zuführen des HF-Stromes zu der Koagulationselektrode 10 des elektrochirurgischen Instruments 40. Der HF-Generator 2 ist mit einer Steuerungseinrichtung 3 zum Steuern der Elektrode 10 des elektrochirurgischen Instruments 40 verbunden. Ein der Steuerungseinrichtung 3 zugeordneter Messwertaufnehmer 4 zur Erfassung verschiedener Parameter, die ein Maß für den Koagulationsgrad darstellen, wie z. B. ein Übergangswiderstand zwischen Elektrode 10 und Gewebe, ist ebenfalls dargestellt.

Das elektrochirurgische Instrument 40 bzw. die Vorrichtung ist mit mindestens einer Elektrode 10 ausgebildet, über die der HF-Koagulationsstrom in das Gewebe einleitbar ist. Die Elektrode 10 ist als dreidimensionaler, auf verschiedene Aufweitungszustände aufweitbarer Körper derart ausgebildet oder auf einem derartigen Körper derart angebracht, dass die Elektrode 10 durch kontinuierliches oder schrittweises Aufweiten des Körpers mit dem Gewebe während des Koagulierens in ständigem elektrischen Kontakt haltbar ist.

Der aufweitbare Elektrodenkörper 10 oder auch ein aufweitbarer Körper 14, auf dem die Elektrode 10 angeordnet ist, ist derart ausgebildet und betätigbar, dass er in das zu behandelnde Gewebe-in nicht aufgeweitetem Zustand einbringbar ist und-während der (insbesondere interstitiellen) Koagulation durch Aufweitung dem behandelten, in der Regel zurückweichenden Gewebe kontrolliert folgt. Dabei kann die Aufweitung des Körpers selbsttätig oder manuell erfolgen, wobei die Aufweitung aufgrund detektierter Parameter, die ein Maß für den Koagulationsgrad darstellen, durchführbar ist. Die kontrollierte Koagulation ermöglicht eine gleichmäßige Gewebedevitalisierung.

Die Vorrichtung zur interstitiellen Koagulation mit dem aufweitbaren Körper 10, 14 ist beispielsweise mittels einer zum Einstechen in das zu behandelnde Gewebe geeigneten Einführeinrichtung (nicht gezeigt) in das Gewebe einbringbar oder aber die Vorrichtung 40 ist selbst derart ausgebildet, dass ein Einstechen in das Gewebe möglich ist. Die Vorrichtung 40 kann sowohl für den unmittelbaren Einsatz oder für die endoskopische Anwendung ausgebildet sein.

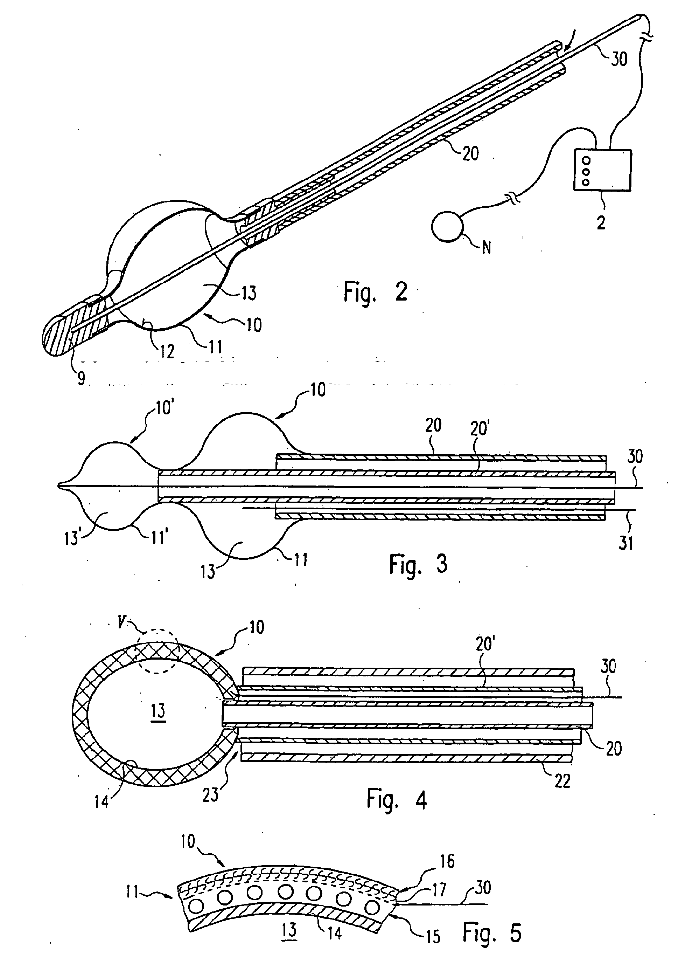

Spezielle Ausgestaltungen der elektrochirurgischen Instrumente 40 und der entsprechenden Elektroden werden in den Fig. 2 bis 5 näher erläutert..

Bei der interstitiellen Koagulation zieht sich das Gewebe aufgrund der Koagulation zusammen, da es austrocknet. Die Elektrode 10 liegt demnach nicht mehr an dem zu behandelnden Gewebe an, so dass aufgrund dieser Spaltbildung ein Übergangswiderstand zwischen Elektrode 10 und Gewebe steigt. Dies hat ein Absinken der Stromstärke des Koagulationsstromes zur Folge.

Die Steuerungseinrichtung 3 ist hier derart ausgelegt, dass sie das Absinken der Stromstärke erfasst und dann das elektrochirurgische Instrument 40 derart ansteuert, dass der Körper 10, 14 zum Nachfolgen des Gewebes aufgeweitet wird. Der Körper 10, 14 wird also in diesem Falle bis zur Überwindung des Spalts aufgeweitet, so dass Elektrode 10 und Gewebe in, dem weiteren Koagulationsvorgang förderlichen, elektrischen Kontakt haltbar sind. Damit ist in jeder Phase der Koagulation ein optimales Koagulationsergebnis gewährleistet. Die erfassten Stromwerte lassen sich z. B. über eine Anzeige 5 ausgeben, so dass ein Operateur den Stromabfall visuell verfolgen kann. Es ist dann mit Hilfe der Anzeige auch möglich, die Aufweitung des Körpers 10, 14 manuell vorzunehmen.

Alternativ ist es möglich, andere, das Koagulationsmaß beschreibende Parameter über den der Steuerungseinrichtung 3 zugeordneten Messwertaufnehmer 4 zu erfassen, beispielsweise den Übergangswiderstand selbst.

Eine beginnende Spaltbildung lässt sich alternativ über eine Druckmessung bestimmen. Dazu ist die Steuerungseinrichtung 3 so ausgebildet, dass beispielsweise der Anpressdruck des Zielgewebes an die Elektrode 10 gemessen wird, wobei bei einem Unterschreiten eines definierten Druckes der Körper 10, 14 derart anzusteuern ist, dass sich dieser zur Wahrung eines geeigneten Abstandes zwischen Elektrode 10 und Gewebe entsprechend aufweitet. Der Messwertaufnehmer 4 ist dazu vorzugsweise als elektronischer Drucksensor ausgebildet. Auch ist es möglich, einen Halleffektsensor zu verwenden, der die Änderung eines Magnetfeldes in Abhängigkeit von der Auslenkung der Elektrode bestimmt.

Die Steuerungseinrichtung 3 kann auch derart angeordnet und ausgebildet sein, dass eine Stromdichte des Koagulationsstromes zwischen Elektrode 10 und Gewebe einstellbar ist. Der dreidimensionale Körper 10, 14 beschreibt in jedem Aufweitungszustand eine definierte Elektrodenfläche. Bei einer konstanten Stromstärke nimmt eine Stromdichte mit zunehmender Aufweitung des Körpers 10, 14 ab, weil sich die Elektrodenfläche vergrößert. Über die Steuerungseinrichtung ist deshalb die für ein optimales Koagulationsergebnis erforderliche Stromdichte einstellbar und die Stromstärke nachregelbar, so dass auch bei sich vergrößernder Elektrodenfläche zu jedem Zeitpunkt des Koagulationsvorganges ein optimales Koagulationsergebnis gewährleistet ist.

Auch hier lässt sich die Einstellung der Stromstärke sowohl selbsttätig als auch manuell durchführen. Da die Stromdichte letztendlich den Aufweitungszustand des Körpers 10, 14 wiedergibt, kann der Chirurg beispielsweise bei nachlassender Stromdichte eine Aufweitung des Körpers 10, 14 veranlassen.

Um unterschiedliche Gewebearten, Koagulationsstadien und/oder unvorhersehbare Ereignisse, wie z. B. eine starke Blutung, berücksichtigen zu können, ist die Steuerungseinrichtung 3 derart ausgebildet, dass die Stromstärke bzw. die Stromdichte unabhängig vom Aufweitungszustand einstellbar ist. Eine unabhängig vom Aufweitungszustand durchgeführte Erhöhung der Stromdichte kann beispielsweise bei Bedarf eine stärkere Koagulation bewirken.

Sind Messeinrichtungen zum Feststellen des Aufweitungszustandes des Körpers 10, 14 vorgesehen, so kann der Aufweitungszustand beispielsweise über die Anzeige 5, vorzugsweise eine optische Anzeige ausgegeben werden. Bei einer manuell durchzuführenden Aufweitung des Körpers 10, 14 kann der Operateur über die Anzeige 5 eine entsprechende Nachregulierung durchführen. Die Messeinrichtungen können über den oben bereits beschriebenen Messwertnehmer realisiert oder aber als eigenständiges Instrument vorgesehen werden.

Die Fig. 2 bis 5 zeigen verschiedene Arten von Vorrichtungen 40 zur interstitiellen Koagulation auf. Diese sind in den Darstellungen derart ausgebildet, dass sie in einen Arbeitskanal eines Endoskops einführbar sind. Das Endoskop muss dann derart ausgebildet sein, dass ein Einstechen in das zu behandelnde Gewebe möglich ist. Die Instrumente können aber auch derart ausgebildet sein, dass sie selbst zum Einstechen geeignet sind.

Wie in Fig. 2 gezeigt, umfasst die Vorrichtung die hier in aufgedehntem Zustand gezeigte Behandlungselektrode 10, welche im Wesentlichen in ihrer Gesamtheit als dehnbares Flächenelement 11 ausgebildet ist. Über einen Zufuhrtubus oder -schlauch 20 kann (mit einem Pfeil angedeutet) elektrisch leitende Flüssigkeit, z. B. Ringer-Lösung oder eine Kochsalz-Lösung durch den Zufuhrschlauch 20 in einen Innenraum 13 der Behandlungselektrode 10 eingefüllt werden. Das dehnbare oder auffaltbare Flächenelement 11 weist hierbei eine Porosität auf, die hinreicht, um in den Innenraum 13 eingeleitete Flüssigkeit von einer Innenseite 12 des dehnbaren Flächenelements 11 herkommend durch die Behandlungselektrode 10 hindurch zur Außenseite austreten zu lassen.

Im Innenraum 13 befindet sich eine drahtförmige Zufuhrelektrode 30, die an einem distalen Ende der Behandlungselektrode 10 über ein elektrisch isolierendes Endstück 9 mechanisch mit der Behandlungselektrode 10 verbunden ist. Die Zufuhrelektrode 30 ist mit dem HF-Generator (hier nicht gezeigt) verbunden.

Um nun eine interstitielle Koagulation durchzuführen, die Elektrode also beispielsweise in einen Tumor einzubringen, wird die gesamte Vorrichtung beispielsweise durch den Arbeitskanal eines Endoskopes, welches bereits bei der Behandlungsstelle platziert wurde, in einem Zustand hindurchgeschoben, in welchem das in Fig. 4 aufgebläht gezeigte Flächenelement noch kollabiert ist. Eine Instrumentenspitze kann so gestaltet sein, dass sie wie ein Dorn durch Verdrängen des Gewebes fortbewegt werden kann. Das ist vorteilhaft, wenn auf dem Weg in das Zielgewebe andere Gewebestrukturen passiert werden müssen, in denen möglichst nur geringer Schaden entstehen soll. Zum Positionieren im Zielgewebe kann es vorteilhaft sein, wenn die Elektrode mit HF-Schneidstrom beaufschlagt wird. Dadurch kann die Gefahr des Freisetzens von Krebszellen beim Einbringen der Elektrode in einen Tumor verringert werden. Ferner kann die Elektrode, weil frei von mechanischer Spannung, exakt positioniert werden. Sobald die Elektrode positioniert ist, wird elektrisch leitende Flüssigkeit (z. B. Ringer-Lösung) in Richtung des in Fig. 2 gezeigten Pfeiles durch den Zufuhrschlauch 20 eingefüllt, wobei der Druck derart eingestellt wird, dass sich die Behandlungselektrode 10 bzw. ihr dehnbares Flächenelement 11 aufblähen lässt. Um den Tumor nun von innen heraus zu koagulieren, wird der HF-Generator 2 betätigt, so dass ein Koagulationsstrom über die Flüssigkeit in das zu behandelnde Gewebe fließt und dieses devitalisiert. Dadurch, dass ständig elektrisch leitfähige Flüssigkeit zugeführt wird und durch das dehnbare Flächenelement 11 austritt, ist gewährleistet, dass ständig ein Flüssigkeitsfilm (oder -polster) zwischen dem dehnbaren Flächenelement 11 und dem zu behandelnden Gewebe verbleibt, durch welchen einerseits der HF-Strom geleitet und andererseits die Oberfläche gekühlt und vor einem Anhaften an der Behandlungselektrode 10 geschützt wird. Das Flächenelement 11 wird im Laufe der Koagulation immer weiter aufgebläht und folgt - wie oben beschrieben - dem zurückweichenden Gewebe.

Bei der oben beschriebenen Ausführungsform der Erfindung wird der HF-Strom monopolar zugeführt. Am Patienten ist also eine Neutralelektrode N als Gegenpol angelegt. Die in Fig. 3 gezeigte Ausführungsform der Erfindung unterscheidet sich nun von der nach Fig. 2 dadurch, dass das Instrument insgesamt bipolar ausgebildet ist. Hierfür sind zwei Behandlungselektroden 10, 10' mit entsprechenden dehnbaren Flächenelementen 11, 11' vorgesehen, die analog der Anordnung nach Fig. 2 (koaxial) aufgebaut sind. Entsprechend den zwei Behandlungselektroden 10, 10' bzw. dehnbaren Flächenelementen 11, 11' sind zwei Zufuhrschläuche 20, 20' sowie zwei Zufuhrelektroden 30, 31 vorgesehen, so dass die Innenräume 13, 13' voneinander unabhängig mit Druck beaufschlagbar und die dehnbaren Flächenelemente 11, 11' unterschiedlich stark dehnbar sind.

Die in Fig. 4 gezeigte Ausführungsform unterscheidet sich insofern von den zuvor beschriebenen Ausführungsformen, als der Innenraum 13 der Behandlungselektrode 10 von einem elastischen, dehnbaren Hilfskörper 14 umschlossen wird, der vollständig dicht ist. Das dehnbare Flächenelement 11 besteht - wie in Fig. 5 gezeigt - aus mehreren Schichten und zwar einer inneren Schicht 15, der über eine Leitung 20' die elektrisch leitende Flüssigkeit in Oberflächenrichtung zugeleitet wird und die in dieser Strömungsrichtung einen relativ niedrigen Strömungswiderstand aufweist. Auf ihrer Außenseite weist die Behandlungselektrode 10 eine äußere Schicht 16 auf, welche die Flüssigkeit vor allem in einer Richtung senkrecht zur Oberfläche leitet. Zwischen der inneren Schicht 15 und der äußeren Schicht 16 ist eine Trennschicht 17 angebracht, welche einen höheren Strömungswiderstand aufweist als die innere Schicht 15, jedoch Flüssigkeit von der inneren Schicht 15 in die äußere Schicht 16 übertreten lässt. Bei der hier gezeigten Ausführungsform der Erfindung ist diese Trennschicht 17 gleichzeitig als elektrisch leitfähige Schicht ausgebildet, welche mit der Zufuhrelektrode 30 in elektrischer Verbindung steht. Bei dieser Anordnung kann somit die Behandlungselektrode 10 bzw. das dehnbare Flächenelement 11 durch den Hilfskörper 14 aufgedehnt werden, wobei zum Aufdehnen nicht nur Flüssigkeit, sondern auch Gas dienen kann. Unabhängig vom Ausdehnungszustand kann dann die elektrisch leitfähige Flüssigkeit zugeführt und über die innere Schicht 15 gleichmäßig verteilt werden. Die austretende Flüssigkeitsmenge bestimmt sich dann ausschließlich durch den Druck, mit welchem die elektrisch leitende Flüssigkeit zugeführt wird, ist also unabhängig vom Dehnungszustand der Behandlungselektrode 10.

Weiterhin ist bei dieser Ausführungsform ein Saugschlauch 22 vorgesehen, der eine Saugöffnung 23 in der Nähe der Behandlungselektrode 10 aufweist. Über diesen Saugschlauch 22 kann überschüssige elektrisch leitende Flüssigkeit abgesaugt werden.

Aus Obigem geht hervor, dass die beschriebenen Merkmale sich auch bei den verschiedenen Ausführungsformen kombinieren lassen. So z. B. kann bei allen Ausführungsformen der Erfindung ein Saugschlauch vorgesehen sein. Ebenso ist es möglich, auch bei der Ausführungsform nach Fig. 2 mit einem Hilfskörper 14 zu arbeiten.

An dieser Stelle sei darauf hingewiesen, dass alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

### Bezugszeichenliste

- 1: HF-Chirurgiegerät
- 2: HF-Generator
- 3: Steuerungseinrichtung
- 4: Messwertnehmer, Messeinrichtung(en)
- 5: Anzeige
- 6: Eingangsanschluss
- 7: Erster Ausgangsanschluss
- 8: Zweiter Ausgangsanschluss
- 9: Endstück
- 10: Elektrode, Behandlungselektrode
- 11: Dehnbares Flächenelement
- 12: Innenseite
- 13: Innenraum
- 14: Hilfskörper
- 8: Zweiter Ausgangsanschluss
- 9: Endstück
- 10: Elektrode, Behandlungselektrode
- 11: Dehnbares Flächenelement
- 12: Innenseite
- 13: Innenraum
- 14: Hilfskörper
- 15: Innere Schicht
- 16: Äußere Schicht
- 17: Trennschicht
- 20: Zufuhrschlauch
- 22: Saugschlauch
- 23: Saugöffnung
- 30: Zufuhrelektrode
- 31: Zufuhrelektrode
- 40: Vorrichtung, elektrochirurgisches Instrument
- N: Neutralelektrode

## Patentansprüche

1. Vorrichtung zur interstitiellen Koagulation von Geweben, mit mindestens einer Elektrode (10), zum Einleiten eines HF-Koagulationsstroms in das Gewebe, wobei die Elektrode (10) als dreidimensionaler, auf verschiedene Aufweitungszustände aufweitbarer Körper ausgebildet oder auf einem derartigen Körper (14) angebracht ist, um die Elektrode (10) durch kontinuierliches oder schrittweises Aufweiten des Körpers (10, 14) mit dem Gewebe während des Koagulierens in ständigem elektrischen Kontakt zu halten,
**gekennzeichnet durch**
eine Steuereinrichtung (3) zum Steuern des Aufweitungszustands des Körpers (10, 14), welche den Körper (10, 14) in Abhängigkeit vom Koagulationsstrom aufweitet.

2. Vorrichtung nach Anspruch 1,
**dadurch gekennzeichnet, dass**
die Steuerungseinrichtung (3) derart angeordnet und ausgebildet ist, dass eine Stromdichte des Koagulationsstromes zwischen Elektrode (10) und Gewebe einstellbar ist.

3. Vorrichtung nach Anspruch 2,
**dadurch gekennzeichnet, dass**
die Steuerungseinrichtung (3) derart ausgebildet ist, dass die Stromdichte unabhängig vom Aufweitungszustand einstellbar ist.

4. Vorrichtung nach einem der vorhergehenden Ansprüche,
**dadurch gekennzeichnet, dass**
Messeinrichtungen (4) zum Feststellen des Aufweitungszustandes des Körpers (10, 14) vorgesehen sind.

5. Vorrichtung nach einem der vorhergehenden Ansprüche,
**dadurch gekennzeichnet, dass**
die Elektrode eine mindestens teilweise fluiddurchlässige Behandlungselektrode (10) umfasst, die mit einem Abschnitt des Gewebes in Berührung bringbar ist, eine Zufuhreinrichtung (20) für Flüssigkeit, über die eine elektrisch leitfähige Flüssigkeit der Behandlungselektrode (10) zuführbar ist, eine Stromzufuhreinrichtung (30, 31) zum Zuführen des HF-Koagulationsstromes zur Behandlungselektrode (10) derart, dass von der Behandlungselektrode (10) durchgelassener Flüssigkeit der HF-Behandlungsstrom zuführbar ist.

6. Vorrichtung nach Anspruch 5,
**dadurch gekennzeichnet, dass**
die Behandlungselektrode (10) ein elastisch dehnbares oder auffaltbares Flächenelement (11) umfasst, auf dessen, dem Gewebe gegenüberliegender Innenseite (12) ein mit einem Innendruck beaufschlagbarer Innenraum (13) derart angeordnet ist, dass das Flächenelement (11) durch Erhöhung des Innendruckes dehnbar ist.

7. Vorrichtung nach Anspruch 6,
**dadurch gekennzeichnet, dass**
das Flächenelement (11) ring- oder kugelförmig ausgebildet ist.

8. Vorrichtung nach einem der Ansprüche 5 - 7,
**dadurch gekennzeichnet, dass**
die Behandlungselektrode (10, 10') in Form eines Ballon-Hohlkatheters aufgebaut ist.

9. Vorrichtung nach einem der Ansprüche 6 - 8,
**gekennzeichnet durch**
Mittel (20) zum Befüllen des Innenraums (13) mit der elektrisch leitfähigen Flüssigkeit.

10. Vorrichtung nach einem der Ansprüche 5 - 9,
**dadurch gekennzeichnet, dass**
die elektrisch leitfähige Flüssigkeit Polyvinylpyrrolidon (PVP), ein Tensid oder dergleichen Mittel zur Veränderung der Viskosität der elektrisch leitfähigen Flüssigkeit umfasst.

11. Vorrichtung nach einem der Ansprüche 5 - 10,
**dadurch gekennzeichnet, dass**
die Behandlungselektrode (10, 10') eine Folie, ein Vlies oder ein Gewebe umfasst und vorzugsweise aus temperaturbeständigem Material, insbesondere aus Tetra-Flour-Ethylen-Material gefertigt ist.

12. Vorrichtung nach einem der vorhergehenden Ansprüche, insbesondere nach einem der Ansprüche 6 - 11,
**dadurch gekennzeichnet, dass**
der Innenraum (13) einen von der elektrisch leitfähigen Flüssigkeit hydraulisch getrennten, expandierbaren Hilfskörper (14) umfasst, wobei vorzugsweise das Flächenelement (11) derart mehrschichtig aufgebaut ist, dass in einer inneren Schicht (15) Flüssigkeit in Flächenrichtung, in einer äußeren Schicht (16) Flüssigkeit senkrecht zur Flächenrichtung leitbar ist, wobei vorzugsweise zwischen der inneren Schicht (15) und der äußeren Schicht (16) eine Trennschicht (17) mit erhöhtem Strömungswiderstand angebracht ist.

13. Vorrichtung nach einem der vorhergehenden Ansprüche,
**gekennzeichnet durch**
eine Saugeinrichtung (22, 23) zum Absaugen von (überschüssiger) Flüssigkeit.

14. Vorrichtung nach einem der vorhergehenden Ansprüche,
**gekennzeichnet durch**
Mittel (30) zum Beaufschlagen der Elektrode (10) mit einem Schneidstrom.

## Claims

1. Appliance for the interstitial coagulation of tissues, with at least one electrode (10) for passing an HF coagulation current into the tissue, wherein the electrode (10) is designed as a three-dimensional body, which can be expanded to different states of expansion, or is mounted on such a body (14), such that the electrode (10) is maintained in constant electrical contact with the tissue, during coagulation, by continuous or stepwise expansion of the body (10, 14), **characterized by** a control device (3) for controlling the state of expansion of the body (10, 14), which control device (3) expands the body (10, 14) in accordance with the coagulation current.

2. Appliance according to Claim 1, **characterized in that** the control device (3) is arranged and designed in such a way that a current density of the coagulation current between electrode (10) and tissue is adjustable.

3. Appliance according to Claim 2, **characterized in that** the control device (3) is designed in such a way that the current density is adjustable independently of the state of expansion.

4. Appliance according to one of the preceding claims, **characterized in that** measurement devices (4) are provided for determining the state of expansion of the body (10, 14).

5. Appliance according to one of the preceding claims, **characterized in that** the electrode comprises a treatment electrode (10), which is at least partially permeable to fluid and which can be brought into contact with a section of the tissue, a delivery device (20) for liquid, via which delivery device (20) an electrically conductive liquid can be delivered to the treatment electrode (10), and a current supply device (30, 31) for supplying the HF coagulation current to the treatment electrode (10), in such a way that the HF treatment current can be supplied to liquid allowed through by the treatment electrode (10).

6. Appliance according to Claim 5, **characterized in that** the treatment electrode (10) comprises an elastically extensible or unfoldable surface element (11), on the inner face (12) of which, opposite the tissue, there is an inner chamber (13) to which an internal pressure can be applied and which is arranged in such a way that the surface element (11) is extensible by increasing the internal pressure.

7. Appliance according to Claim 6, **characterized in that** the surface element (11) has a ring-shaped or ball-shaped design.

8. Appliance according to one of Claims 5-7, **characterized in that** the treatment electrode (10, 10') is constructed in the form of a balloon catheter.

9. Appliance according to one of Claims 6-8, **characterized by** means (20) for filling the inner chamber (13) with the electrically conductive liquid.

10. Appliance according to one of Claims 5-9, **characterized in that** the electrically conductive liquid comprises polyvinylpyrrolidone (PVP), a surfactant or similar agent for changing the viscosity of the electrically conductive liquid.

11. Appliance according to one of Claims 5-10, **characterized in that** the treatment electrode (10, 10') comprises a film, a nonwoven or a woven, and is preferably made of temperature-resistant material, in particular of tetrafluoroethylene material.

12. Appliance according to one of the preceding claims, in particular according to one of Claims 6-11, **characterized in that** the inner chamber (13) comprises an expandable auxiliary body (14) which is hydraulically separated from the electrically conductive liquid, and the surface element (11) is preferably constructed in several layers, in such a way that, in an inner layer (15), liquid can be conveyed in a surface direction and, in an outer layer (16), liquid can be conveyed in a direction perpendicular to the surface direction, wherein a separating layer (17) with greater current resistance is preferably placed between the inner layer (15) and the outer layer (16).

13. Appliance according to one of the preceding claims, **characterized by** a suction device (22, 23) for sucking off (excess) liquid.

14. Appliance according to one of the preceding claims, **characterized by** means (30) for supplying the electrode (10) with a cutting current.

## Revendications

1. Dispositif pour la coagulation interstitielle de tissus, avec au moins une électrode (10), destinée à introduire un courant de coagulation HF dans le tissu, dans lequel l'électrode (10) est réalisée sous la forme d'un corps tridimensionnel, extensible à différents états d'élargissement, ou est appliquée sur un tel corps (14), afin de maintenir l'électrode (10) en contact électrique permanent avec le tissu pendant la coagulation par un élargissement continu ou progressif du corps (10, 14), **caractérisé par** un dispositif de commande (3) destiné à commander l'état d'élargissement du corps (10, 14), qui élargit le corps (10, 14) en fonction du courant de coagulation.

2. Dispositif selon la revendication 1, **caractérisé en ce que** le dispositif de commande (3) est disposé et conçu de telle manière qu'une densité de courant du courant de coagulation entre l'électrode (10) et le tissu soit réglable.

3. Dispositif selon la revendication 2, **caractérisé en ce que** le dispositif de commande (3) est conçu de telle manière que la densité de courant soit réglable indépendamment de l'état d'élargissement.

4. Dispositif selon l'une quelconque des revendications précédentes, **caractérisé en ce qu'**il est prévu des dispositifs de mesure (4) pour constater l'état d'élargissement du corps (10, 14).

5. Dispositif selon l'une quelconque des revendications précédentes, **caractérisé en ce que** l'électrode comprend une électrode de traitement (10) au moins partiellement perméable au fluide, qui peut être mise en contact avec une partie du tissu, un dispositif d'arrivée (20) pour le liquide, par lequel un liquide électriquement conducteur peut être amené à l'électrode de traitement (10), un dispositif d'arrivée de courant (30, 31) pour amener le courant de coagulation HF à l'électrode de traitement (10), de telle manière que le courant de traitement HF puisse être amené au liquide écoulé par l'électrode de traitement (10).

6. Dispositif selon la revendication 5, **caractérisé en ce que** l'électrode de traitement (10) comprend un élément plat (11) élastiquement extensible ou repliable, dont le côté intérieur (12), faisant face au tissu, définit un espace intérieur (13) pouvant être soumis à une pression interne, de telle manière que l'élément plat (11) soit extensible par augmentation de la pression interne.

7. Dispositif selon la revendication 6, **caractérisé en ce que** l'élément plat (11) est réalisé en forme d'anneau ou de sphère.

8. Dispositif selon l'une quelconque des revendications 5 à 7, **caractérisé en ce que** l'électrode de traitement (10, 10') est construite sous la forme d'un cathéter creux à ballonnet.

9. Dispositif selon l'une quelconque des revendications 6 à 8, **caractérisé par** des moyens (20) pour remplir l'espace intérieur (13) avec le liquide électriquement conducteur.

10. Dispositif selon l'une quelconque des revendications 5 à 9, **caractérisé en ce que** le liquide électriquement conducteur comprend de la polyvinylpyrrolidone (PVP), un agent tensioactif ou des moyens similaires pour modifier la viscosité du liquide électriquement conducteur.

11. Dispositif selon l'une quelconque des revendications 5 à 10, **caractérisé en ce que** l'électrode de traitement (10, 10') comprend une feuille, un non-tissé ou un tissu et est fabriquée de préférence en un matériau résistant à la température, en particulier en matériau de tétrafluoroéthylène.

12. Dispositif selon l'une quelconque des revendications précédentes, en particulier selon l'une quelconque des revendications 6 à 11, **caractérisé en ce que** l'espace intérieur (13) comprend un corps auxiliaire expansible (14), hydrauliquement séparé du liquide électriquement conducteur, dans lequel l'élément plat (11) est de préférence composé de plusieurs couches, de telle manière que du liquide puisse être guidé dans une couche intérieure (15) dans la direction du plat et que du liquide puisse être guidé dans une couche extérieure (16) perpendiculairement à la direction du plat, dans lequel une couche de séparation (17) présentant une résistance hydraulique accrue est de préférence disposée entre la couche intérieure (15) et la couche extérieure (16).

13. Dispositif selon l'une quelconque des revendications précédentes, **caractérisé par** un dispositif d'aspiration (22, 23) destiné à aspirer le liquide (en excès).

14. Dispositif selon l'une quelconque des revendications précédentes, **caractérisé par** des moyens (30) pour appliquer à l'électrode (10) un courant de coupe.
